(19) Europäisches Patentamt
European Patent Office
Office européen des brevets

(11) Numéro de publication: **0 069 002**
**B1**

# (12) FASCICULE DE BREVET EUROPEEN

(45) Date de publication du fascicule du brevet: **04.09.85**

(51) Int. Cl.⁴: **C 07 D 333/20** // C07D333/22

(21) Numéro de dépôt: **82401132.4**

(22) Date de dépôt: **21.06.82**

## (54) Procédé de préparation de (thiényl-2)-2 éthylamines et (thiényl-3)-2 éthylamines.

(30) Priorité: **30.06.81 FR 8113066**

(43) Date de publication de la demande:
**05.01.83 Bulletin 83/1**

(45) Mention de la délivrance du brevet:
**04.09.85 Bulletin 85/36**

(84) Etats contractants désignés:
**AT BE CH DE FR GB IT LI LU NL SE**

(56) Documents cités:
**DE - A - 2 623 174**

**THE JOURNAL OF ORGANIC CHEMISTRY, vol.15, no.4, juillet 1950, (US) R.T. GILSDORF et al.: "Studies on the chemistry of heterocyclics. IX. Reduction of thienyl nitroolefins with lithium aluminium hydride", pages 807-811**
**CHEMICAL ABSTRACTS, vol.67, no.15, 9 octobre 1967, page 6913, résumé no.73465y, Columbus, Ohio (US) L.K. FREIDLIN et al.: "Hydrogenation of heterocyclic analogs of bêta-nitrostyrene on pdcatalyst"**

(73) Titulaire: **SANOFI, Société dite:, 40, Avenue George V, F-75008 Paris (FR)**

(72) Inventeur: **Anne-Archard, Gilles, 8 Rue Dupont, F-31500 Toulouse (FR)**
Inventeur: **Heymes, Alain, Chemin de l'Adrech, F-04200 Sisteron (FR)**
Inventeur: **Valette, Gérard, "La Riverotte" Clermont Le Fort, F-31120 Portet/Garonne (FR)**

(74) Mandataire: **Bressand, Georges et al, c/o CABINET LAVOIX 2 Place d'Estienne d'Orves, F-75441 Paris Cedex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

La présente invention est relative à un nouveau procédé de préparation de (thiényl-2)-2 éthylamines et (thiényl-3)-2 éthylamines composés, dont un nombre important sont connus et utilisés comme intermédiaires pour l'obtention de dérivés employés aussi bien dans l'industrie chimique que pharmaceutique.

Les composés préparés suivant le procédé de l'invention répondent à la formule I

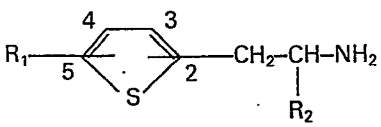 (I)

dans laquelle $R_1$ et $R_2$ représentent un atome d'hydrogène ou un radical alkyle linéaire ou ramifié ou un radical aromatique hétérocyclique ou non tel que thiényle, furyle, pyridyle, phényle, naphtyle éventuellement mono- ou polysubstitué.

La chaîne aminoéthyle et le groupe $R_1$ peuvent occuper n'importe quelle position sur le noyau thiophénique.

Les dérivés de formule I ont déjà été préparés selon différentes méthodes:

– réduction de béta-nitrovinyl-2 ou -3 thiophènes à l'aide d'hydrure double de lithium et d'aluminium [R.T. GILSDORF et F.F. NORD, J. Org. Chem., 15, 807 (1950); E. CAMPAIGNE et W.C. Mc. CARTHY, J. Am. Chem. Soc., 76, 4466 (1954)] ou par voie électrochimique [FR. 7801992];

– réduction de [thiényl-2]- et [thiényl-3]-2 acétonitriles à l'aide d'hydrure double de lithium et d'aluminium [B.F. CROWE et F.F. NORD, J. Org. Chem., 15, 81 (1950); E. CAMPAIGNE et W.C. Mc. CARTHY, réf. citée] ou de l'amalgame de sodium [F.F. BLICKE et J.H. BURCKHALTER, J. Am. Chem. Soc., 64, 477 (1942)];

– par dégradation de Curtius sur les acides (thiényl-2)- et (thiényl-3)-3 propioniques [G. BARGER et A.P.T. EASSOU, J. Chem. Soc., 2100 (1938); E. CAMPAIGNE et W.C. Mc. CARTHY, réf. citée];

– à partir de dérivés (halogénures ou arylsulfonates) de (thiényl-2)- et (thiényl-3)-2 éthanol par emination directe [F.F. BLICKE et J.H. BURCKHALTER, réf. citée; FR. 7503142] ou par l'intermédiaire d'un phtalimide [FR. 7503142].

Mais ces différents procédés, difficiles à mettre en œuvre, ne permettaient pas d'obtenir de bons rendements, interessants sur le plan industriel.

L'objet de la présente invention est donc de permettre de préparer, par un procédé simple et peu onéreux, les composés de formule I.

Pour parvenir à ce but, on met en œuvre, au départ, un dérivé de formule IV

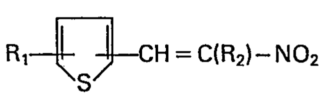 (IV)

dans laquelle $R_1$ et $R_2$ sont définis tels que dans la formule I et que l'on peut préparer par diverses méthodes bien connues de l'homme de l'art telle que par exemple la condensation d'un composé de formule II

$$R_2-CH_2-NO_2 \qquad (II)$$

dans laquelle $R_2$ est également défini tel que dans la formule I, avec un dérivé carbonylé de formule III

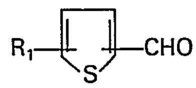 (III)

dans laquelle $R_1$ est également défini tel que dans la formule I, en opérant dans des conditions bien connues de l'homme de l'art pour obtenir un dérivé de formule IV

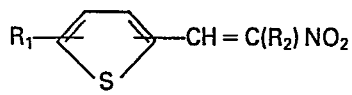 (IV)

Conformément au procédé selon l'invention pour préparer les composés de formule I

a) on soumet à une réaction d'hydrogénation catalytique le dérivé de formule IV qui, dans les conditions mises en œuvre, conduit à un composé de formule V

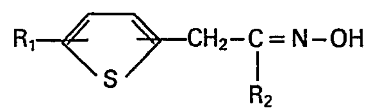 (V)

b) le composé est, à son tour, transformé également par hydrogénation catalytique mais dans des conditions opératoires différentes en dérivé de formule I tel que défini plus haut.

D'une manière générale, l'obtention de composés du type arylétylamino selon le schéma cidessous

$$Ar-CH=\underset{\displaystyle R}{\underset{|}{C}}-NO_2 \xrightarrow[Cat]{H_2} Ar-CH_2-\underset{\displaystyle R}{\underset{|}{CH}}-NH_2$$

c'est-à-dire par hydrogénation catalytique de béta-nitrolvinylaryl est une réalité déjà connue. Toutefois, elle n'a jamais pu être appliquée dans les cas où le radical aromatique est le thiophène. Néanmoins, il a déjà été décrit une certaine hydrogénation du béta-nitro-vinyl-2 thiophène limitée toutefois à l'obtention de l'oxime du (thiényl-2)-2 acétaldéhyde réalisée en présence de palladium [L. Kh. FREIDLIN, E.F. LITVIN et V.M. CHURSINA, Khimiya' G. et Sced., 3, 22 (1967), Chem. Abst., 67, 73465 y] mais les quantités de catalyseur mises en œuvre (0,200 g de palladium métallique pour 0,775 g de substrat à hydrogéner) excluent toute application industrielle raisonnalbe: la proportion de palladium utilisée est en effet supérieure à 25% par rapport au substrat.

Au contraire, par le procédé de l'invention on aboutit aux (thiényl-2)- ou aux (thiényl-3)-2 éthylamines dans des conditions faciles à mettre en

œuvre et peu coûteuses du fait des faibles quantités de catalyseur utilisées.

Elle se caractérise notamment par le fait que l'on opère cette réduction en deux temps.

En un premier temps, le dérivé de formule IV est hydrogéné en milieu non alcalin

– au sein d'un solvant qui peut être un alcool tel que, par exemple, le méthanol, l'éthanol, l'isopropanol ou un acide organique tel que, par exemple, l'acide formique, l'acide acétique, l'acide propionique ou plus avantageusement encore, au sein d'un mélange de deux solvants choisis chacun dans l'une des familles mentionnées comme, par exemple, un mélange de méthanol ou d'éthanol et d'acide acétique,

– en présence d'un catalyseur métallique qui peut être le palladium, le platine, le ruthénium, le rhodium ou l'iridium.

Le métal est avantageusement dilué avec 10 à 100 fois son poids d'un support comme, par exemple, le charbon, l'alumine ou le sulfate de baryum et, généralement, on met en œuvre une quantité de cet ensemble comprise entre 1 et 10% du substrat à hydrogéner, ce qui correspond à des quantités de 1/10.000 à 1/100 de palladium

– sous une pression d'hydrogène généralement comprise entre 1 et 100 atmosphères, plus particulièrement entre 5 et 25 atmosphères,

– à une température généralement comprise entre 20 et 100°C plus particulièrement entre 30 et 60°C,

– la durée de l'opération est généralement comprise entre 30 minutes et quelques heures.

En un second temps, le dérivé de formule V isolé du milieu réactionnel par des moyens appropriés, après séparation du catalyseur, est hydrogéné

– au sein d'un solvant qui peut être un alcool comme, par exemple, le méthanol, l'éthanol ou l'isopropanol qui avantageusement, pour éviter la formation d'amines secondaires, contiendra de l'ammoniac à l'état dissous,

– en présence d'un catalyseur métallique et tout particulièrement de nickel qu'il s'agisse de nickel dit de Raney ou de nickel disposé sur un support comme la silice ou l'alumine, la quantité de métal mise en œuvre étant comprise entre 1 et 10% du substrat à hydrogéner,

– sous une pression d'hydrogène généralement comprise entre 1 et 100 atmosphères plus particulièrement entre 5 et 25 atmosphères,

– à une température généralement comprise entre 20 et 100°C, plus particulièrement entre 40 et 80°C,

– la durée de l'opération est généralement comprise entre 30 minutes et quelques heures.

Les composés de formule I ainsi obtenus peuvent ensuite être isolés et purifiés selon les méthodes habituelles. Pour réaliser ces opérations, il peut être avantageux de transformer les composés libres de formule I en leurs sels, par exemple en leurs sels d'addition d'acides par réaction avec des acides minéraux ou organiques. A partir des sels, on peut libérer les composés de formule I selon les méthodes connues.

Les exemples suivants illustrent l'invention sans la limiter.

Exemple 1

Préparation du chlorhydrate de (thiényl-2)-éthylamine

a) Oximes du thiényl-2, acétaldéhyde

100 g (0,645 mole) de (thiényl-2)-2 nitroéthylène en solution dans 2 litres d'un mélange d'acide acétique et d'éthanol (75/25) sont hydrogénés en autoclave à 35°C sous une pression de 20 bars en présence de 5g de palladium sur charbon à 5% jusqu'à refus d'absorption (durée: environ deux heures). Après filtration du catalyseur et rinçage de ce dernier avec de l'éthanol, la solution obtenue est évaporée sous vide à 50°C. Le résidu huileux est repris par 500 ml de chlorure de méthylène et la solution est lavée successivement avec une solution aqueuse environ N de soude puis à l'eau et finalement évaporée pour fournir 97g d'oximes (mélange syn + anti) du thiényl-2, acétaldéhyde sous forme d'une huile jaune orangée engagée telle quelle dans le stade suivant.

b) Le mélange d'oximes obtenu ci-dessus, en solution dans 2 litres de méthanol saturé en ammoniac est hydrogéné en autoclave à 60°C sous une pression de 20 bars en présence de 10 g de nickel de Raney jusqu'à refus d'absorption (durée: environ 20 heures). Après filtration du catalyseur et rinçage de ce dernier avec du méthanol, la solution obtenue est évaporée. Le résidu huileux est additionné de 500 ml de toluène. Cette phase organique est extraite avec une solution aqueuse environ N d'acide chlorhydrique. La phase aqueuse acide isolée est basifiée par la soude aqueuse environ N puis extraite au toluène. L'addition à la phase organique isolée d'une solution d'acide chlorhydrique gazeux dans l'éther isopropylique entraîne la formation d'un précipité qui est filtré puis séché. On obtient ainsi 68 g (rendement 65% par rapport au (thiényl-2)-2 nitroéthylène) de chlorhydrate de (thiényle-2)-2 éthylamine sous forme de cristaux blancs.

F = 202°C.

Analyse $C_6H_9NS$, HCl = 163,67

| | | | |
|---|---|---|---|
| Calculé: | C % 44,03 | H % 6,15 | N % 8,55 |
| Trouvé: | 43,98 | 6,16 | 8,54. |

Exemple 2

Chlorhydrate de (thiényl-3)-2 éthylamine

En opérant dans les conditions décrites à l'exemple 1, mais en substituant le Rd/C à 5% au Pd/C à 5%, on prépare avec 66% de rendement le chlorhydrate du composé.

F = 216°C.

Analyse $C_6H_9NS$, HCl = 163,67

| | | | |
|---|---|---|---|
| Calculé: | C % 44,03 | H % 6,15 | N % 8,55 |
| Trouvé: | 43,92 | 6,18 | 8,50. |

Exemple 3

Chlorhydrate de la méthyl-1, (thiényl-3)-2 éthylamine

En opérant dans les conditions telles que décrites à l'exemple 1, mais en substituant le Pt/C à

5% au Pd/C à 5%, on prépare avec 81% de rendement le chlorhydrate du composé.

F = 138°C.

Analyse $C_7H_{11}NS$, HCl = 177,69

| | | | |
|---|---|---|---|
| Calculé: | C % 44,31 | H % 6,80 | N % 7,88 |
| Trouvé: | 47,55 | 6,85 | 7,92. |

**Revendications**

1. Procédé de préparation de (thiényl-2)-2 et (thiényl-3)-2 éthylamines de formule I

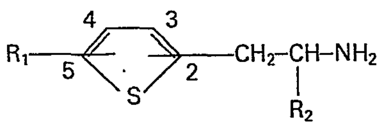
(I)

dans laquelle $R_1$ et $R_2$ représentent un atome d'hydrogène, un radical alcoyle linéaire ou ramifié, un radical aromatique hétérocyclique ou non, éventuellement mono- ou polysubstitué, caractérisé en ce que l'on soumet à une hydrogénation catalytique, en milieu non alcalin dans un alcool ou un acide organique ou un mélange des deux sous une pression d'hydrogène comprise entre 1 et 100 atmosphères, à une température comprise entre 20 et 100°C, et en présence d'un catalyseur métallique associé à un support inerte, un composé de formule IV

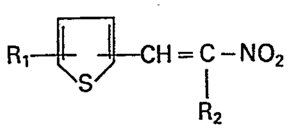
(IV)

dans lequel $R_1$ et $R_2$ sont tels que définis précédemment, pour obtenir le composé de formule V

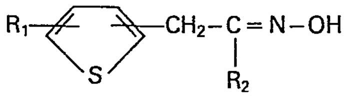
(V)

dans laquelle $R_1$ et $R_2$ sont tels que définis précédement, et qui, après isolement à son tour est transformé, par hydrogénation catalytique, en milieu alcalin, sous une pression d'hydrogène comprise entre 1 et 100 atmosphères, à une température comprise entre 20 et 100°C, et en présence d'un catalyseur métallique sur un support, en un composé de formule I.

2. Procédé suivant la revendication 1, caractérisé en ce que la première étape d'hydrogénation du composé est effectuée dans un mélange d'un alcool et d'un acide organique.

3. Procédé suivant la revendication 1, caractérisé en ce que le catalyseur métallique de la première étape d'hydrogénation est chosi parmi le palladium, le platine, le ruthénium, le rhodium et l'iridium, associé à un support inerte.

4. Procédé selon la revendication 3, caractérisé en ce que le support inerte est le charbon, l'alumine ou le sulfate de baryum.

5. Procédé selon la revendication 1, caractérisé en ce que la première étape d'hydrogénation est effectuée sous une pression d'hydrogène comprise entre 5 et 25 atmosphères.

6. Procédé selon la revendication 1, caractérisé en ce que la première étape d'hydrogénation est effectuée à une température comprise entre 30 et 60°C.

7. Procédé suivant la revendication 4, caractérisé en ce que le constituant métallique du catalyseur est utilisé dans des proportions variant de 1/10.000 à 1/100 par rapport au substrat à hydrogéner.

8. Procédé selon la revendication 1, caractérisé en ce que le catalyseur de la seconde étape d'hydrogénation du composé V est le nickel Raney ou un catalyseur comprenant du nickel déposé sur un support inerte.

9. Procédé selon la revendication 8, caractérisé en ce que la constituant métallique du catalyseur est utilisé à raison de 1 à 10% par rapport au substrat.

10. Procédé selon la revendication 1, caractérisé en ce que la seconde étape d'hydrogénation est effectuée sous une pression d'hydrogène comprise entre 5 et 25 atmosphères.

11. Procédé selon la revendication 1, caractérisé en ce que la seconde étape d'hydrogénation est effectuée à une température comprise entre 40 et 80°C.

12. Procédé suivant la revendication 8, caractérisé en ce que l'hydrogénation est effectuée dans un alcool, en association avec de l'ammoniac à l'état dissous.

13. Procédé suivant la revendication 12, caractérisé en ce que l'alcool est le méthanol, l'éthanol ou l'isopropanol.

14. Procédé selon la revendication 1, caractérisé en ce que le radical aromatique est choisi parmi les groupes thiényle, furyle, pyridyle, phényle ou naphtyle.

**Patentansprüche**

1. Verfahren zur Herstellung von (2-Thienyl)- und 2-(3-Thienyl)-ethylaminen der Formel I

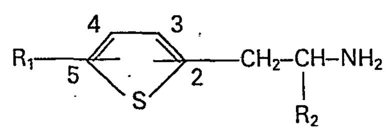
(I)

worin $R_1$ und $R_2$ ein Wasserstoffatom, einen linearen oder verzweigten Alkylrest oder einen heterocyclischen oder nicht-heterocyclischen, gegebenenfalls einfach oder mehrfach substituierten aromatischen Rest darstellen, dadurch gekennzeichnet, dass man eine Verbindung der Formel IV

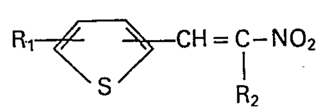
(IV)

worin $R_1$ und $R_2$ die vorstehend genannten Bedeutungen haben, einer katalytischen Hydrierung in einem nicht-alkalischen Medium in einem Alkohol oder einer organischen Säure oder einem Gemisch der beiden unter einem Wasserstoffdruck,

der zwischen 1 und 100 Atm liegt, bei einer Temperatur, die zwischen 20 und 100°C liegt, in Gegenwart eines metallischen Katalysators, der mit einem inerten Träger verbunden ist, unterwirft, um die Verbindung der Formel V

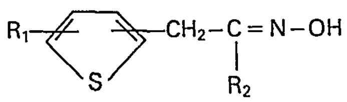 (V)

zu erhalten, worin $R_1$ und $R_2$ die vorstehend genannten Bedeutungen haben, zu erhalten, die nach Isolierung ihrerseits durch katalytische Hydrierung in einem alkalischen Medium unter einem zwischen 1 und 100 Atm liegenden Wasserstoffdruck bei einer zwischen 20 und 100°C liegenden Temperatur in Gegenwart eines metallischen Katalysators auf einem Träger in eine Verbindung der Formel I umgewandelt wird.

2. Verfahren nach Anspruch 1, dadurch gekennzeichnet, dass die erste Stufe der Hydrierung der Verbindung IV in einem Gemisch eines Alkohols und einer organischen Säure durchgeführt wird.

3. Verfahren nach Anspruch 1, dadurch gekennzeichnet, dass der mit einem inerten Träger verbundene metallische Katalysator der ersten Hydrierstufe aus Palladium, Platin, Ruthenium, Rhodium und Iridium ausgewählt ist.

4. Verfahren nach Anspruch 3, dadurch gekennzeichnet, dass der inerte Träger Kohle, Aluminiumoxid oder Bariumsulfat ist.

5. Verfahren nach Anspruch 1, dadurch gekennzeichnet, dass die erste Hydrierstufe unter einem zwischen 5 und 25 Atm liegenden Wasserstoffdruck durchgeführt wird.

6. Verfahren nach Anspruch 1, dadurch gekennzeichnet, dass die erste Hydrierstufe bei einer zwischen 30 und 60°C liegenden Temperatur durchgeführt wird.

7. Verfahren nach Anspruch 4, dadurch gekennzeichnet, dass der metallische Bestandteil des Katalysators in Anteilen, die von 1/10000 bis 1/100, bezogen auf das zu hydrierende Substrat, variieren, verwendet wird.

8. Verfahren nach Anspruch 1, dadurch gekennzeichnet, dass der Katalysator der zweiten Stufe der Hydrierung der Verbindung V Raney-Nickel oder ein Katalysator ist, der auf einen inerten Träger aufgebrachtes Nickel enthält.

9. Verfahren nach Anspruch 8, dadurch gekennzeichnet, dass der metallische Bestandteil des Katalysators in einer Menge von 1 bis 10%, bezogen auf das Substrat, verwendet wird.

10. Verfahren nach Anspruch 1, dadurch gekennzeichnet, dass die zweite Hydrierstufe unter einem zwischen 5 und 25 Atm liegenden Wasserstoffdruck durchgeführt wird.

11. Verfahren nach Anspruch 1, dadurch gekennzeichnet, dass die zweite Hydrierstufe bei einer zwischen 40 und 80°C liegenden Temperatur durchgeführt wird.

12. Verfahren nach Anspruch 8, dadurch gekennzeichnet, dass die Hydrierung in einem Alkohol in Verbindung mit Ammoniak im gelösten Zustand durchgeführt wird.

13. Verfahren nach Anspruch 12, dadurch gekennzeichnet, dass der Alkohol Methanol, Ethanol oder Isopropanol ist.

14. Verfahren nach Anspruch 1, dadurch gekennzeichnet, dass der aromatische Rest aus den Gruppen Thienyl, Furyl, Pyridyl, Phenyl oder Naphthyl ausgewählt ist.

**Claims**

1. Process for the preparation of 2-(2-thienyl)- and -(3-thienyl -ethylamines of the formula I:

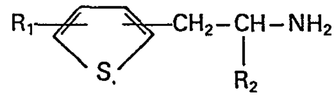 (I)

in which $R_1$ and $R_2$, which may be the same or different, represent each a hydrogen atom, a straight- or branched-chain alkyl radical, a heterocyclic or non-heterocyclic aromatic radical, optionally mono- or poly-substituted, comprising catalytically hydrogenating in non alcaline medium in an alcohol or an organic acid or their mixture under a hydrogen pressure of 1 to 100 atmospheres at a temperature of 20 to 100°C, and in the presence of a metal catalyst associated with an inert carrier, a compound of the formula IV:

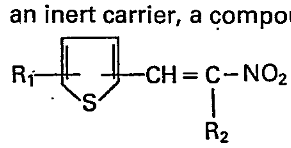 (IV)

in which $R_1$ and $R_2$ are as defined above, to give a compound of the formula V:

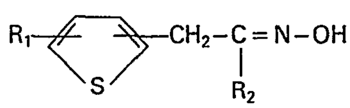 (V)

in which $R_1$ and $R_2$ are as defined above, isolating compound (V) and then catalytically hydrogenating same in alcaline medium under a hydrogen pressure of 1 to 100 atmospheres, at a temperature of 20 to 100°C, and in the presence of a metal catalyst on a carrier to give a compound of the formula (I).

2. Process as claimed in claim 1, wherein the first step for hydrogenating compound IV is carried out within a mixture of an alcohol and an acid.

3. Process as claimed in claim 1, wherein the metal catalyst of the first hydrogenation step is selected from palladium, platinum, ruthenium, rhodium and iridium, associated with an inert carrier.

4. Process as claimed in claim 3, wherein the inert carrier is charcoal, alumina or baryum sulfate.

5. Process as claimed in claim 1, wherein the first hydrogenation step is carried out under a hydrogen pressure of 5 to 25 atmospheres.

6. Process as claimed in claim 1, wherein the first hydrogenation step is carried out at a temperature of 30 to 60°C.

7. Process as claimed in claim 4, wherein the metal component of the catalyst is used in a ratio of 1/10,000 to 1/100 with respect to the substrate.

8. Process as claimed in claim 1, wherein the catalyst of the second hydrogenation step of compound V is a nickel Raney catalyst or a catalyst comprising nickel deposited on an inert carrier.

9. Process as claimed in claim 8, wherein the metal component of the catalyst is used in an amount of 1 to 10% with respect to the substrate.

10. Process as claimed in claim 1, wherein the second hydrogenation step is carried out under a hydrogen pressure of 5 to 25 atmospheres.

11. Process as claimed in claim 1, wherein the second hydrogenation step is carried out at a temperature of 40 to 80°C.

12. Process as claimed in claim 8, wherein the reaction is carried out within an alcohol, in combination with dissolved ammonia.

13. Process according to claim 12, wherein the alcohol is methanol, ethanol or isopropanol.

14. Process as claimed in claim 1, wherein the aromatic radical is selected from thienyl, furyl, pyridyl, phenyl and naphthyl.